# FASCICULE DE BREVET EUROPEEN

(11) **EP 3 263 542 B1**
(45) Date de publication et mention de la délivrance du brevet: **13.11.2019**
(21) Numéro de dépôt: 17180420.6
(22) Date de dépôt: 11.01.2013
(51) Int. Cl.: C07C 17/20, C07C 17/25, C07C 17/383, C07C 21/18

(54) **PROCEDE DE PRODUCTION DE 2,3,3,3-TETRAFLUOROPROPENE**
HERSTELLUNGSVERFAHREN VON 2,3,3,3-TETRAFLUORPROPEN
METHOD FOR PRODUCING 2,3,3,3-TETRAFLUOROPROPENE

(30) Priorité: 03.02.2012 FR 1251021
(43) Date de publication de la demande: 03.01.2018
(62) Demande divisionnaire de: 13701863.6
(73) Titulaire: ARKEMA FRANCE, 92700 Colombes (FR)
(72) Inventeur: WENDLINGER, Laurent, 69510 SOUCIEU EN JARREST (FR); COLLIER, Bertrand, 69230 SAINT-GENIS-LAVAL (FR); DEUR-BERT, Dominique, 69390 CHARLY (FR)
(74) Mandataire: Leca, François Michel

(56) Documents cités:
- WO-A1-2011/077192
- US-A1- 2009 270 659

## Description

### DOMAINE DE L'INVENTION

La présente invention concerne un procédé de production de 2,3,3,3-tétrafluoropropène (HFO-1234yf), notamment à partir de 1,1,1,2,3-pentachloropropane (HCC-240db) et / ou de 1,1,2,2,3-pentachloropropane (HCC-240aa), ainsi qu'une installation adaptée à la mise en œuvre de ce procédé.

### ARRIERE-PLAN TECHNIQUE

Le protocole de Montréal pour la protection de la couche d'ozone a conduit à l'abandon de l'utilisation des chlorofluorocarbures (CFC). Des composés moins agressifs pour la couche d'ozone, tels que les hydrofluorocarbures (HFC) ont ainsi remplacé les chlorofluorocarbures. Toutefois ces composés contribuent de manière relativement importante à l'effet de serre. Il existe donc un besoin de composés efficaces présentant à la fois un faible coefficient ODP (potentiel de déplétion de l'ozone) et un faible coefficient GWP (potentiel de réchauffement climatique). Les hydrofluorooléfines (HFO) ont été identifiées comme des alternatives souhaitables, du fait de leurs faibles valeurs d'ODP et de GWP.

Le composé 2,3,3,3-tétrafluoropropène (HFO-1234yf) est particulièrement intéressant à cet égard.

Plusieurs documents concernent des procédés de préparation d'oléfines fluorées à partir de pentachloropropane.

Par exemple, le document US 2009/0240090 décrit la réaction du 1,1,1,2,3-pentachloropropane (HCC-240db) en 2-chloro-3,3,3-trifluoropropène (HCFO-1233xf) en l'absence d'oxygène. Le HCFO-1233xf ainsi produit est converti en 2-chloro-1,1,1,2-tétrafluoropropane (HCFC-244bb).

Le document WO 2009/015317 décrit la réaction d'un composé chloré tel que le 1,1,2,3-tétrachloropropène (HCO-1230xa), le HCC-240db ou le 2,3,3,3-tétrachloropropène (HCO-1230xf) avec du fluorure d'hydrogène (HF), en phase gazeuse. Ce procédé permet d'obtenir du HCFO-1233xf.

Le document WO 2005/108334 décrit la réaction du HCC-240db avec du HF pour donner du 2-chloro-1,1,1,3-tétrafluoropropane (HCFC-244db), qui est ensuite déhydrochloré pour fournir du 1,3,3,3-tétrafluoropropène (HFO-1234ze).

Le document WO 2010/123148 décrit la fluoration du HCC-240db en HCFO-1233xf en l'absence de catalyseur.

Le document WO2011/077192 décrit la fluoration du 2-chloro-3,3,3-trifluoropropène en 2,3,3,3-tétrafluoropropène au moyen de fluorure d'hydrogène.

La demande PCT/IB2010/003028, déposée par le demandeur, décrit la fluoration catalytique en phase gazeuse du HCC-240db ou du 1,1,2,2,3-pentachloropropane (HCC-240aa) en HFO-1234yf.

Toutefois, dans le cadre de ce procédé, il est nécessaire de séparer les composés issus de l'étape de réaction, à savoir le produit recherché HFO-1234yf, d'autres composés fluorés formés lors de la réaction, l'acide chlorhydrique (HCl) également formé lors de la réaction, les réactifs non réagis (notamment HF), des produits de dégradation ou des composés inertes.

Pour ce faire, on peut utiliser un ensemble de colonnes de distillation permettant d'obtenir un flux de HFO-1234yf, un flux de HCl et un flux destiné à être recyclé à la réaction. Or les points d'ébullition du HCl et du HFO-1234yf sont relativement bas (-85°C pour le HCl à pression atmosphérique et -29,1°C pour le HFO-1234yf à pression atmosphérique). La condensation des gaz en tête des colonnes de distillation nécessite donc une pression d'opération élevée, afin que la température nécessaire en tête de colonne ne soit pas trop basse, et donc afin que le procédé soit compatible avec l'utilisation de groupes froids standards.

Typiquement, la pression d'opération nécessaire pour la séparation par distillation est supérieure à 5 bar, voire supérieure à 7 bar. Le réacteur doit donc être opéré à une pression encore supérieure. Cela pose problème, dans la mesure où, dans certaines configurations, il est souhaitable d'opérer à une pression relativement faible dans le réacteur.

Si la réaction de fluoration est effectuée à plus faible pression, les gaz issus de la réaction doivent être comprimés avant la distillation. Toutefois, cela implique une taille de compresseur excessive, en raison de l'excès important de HF utilisé dans la réaction de fluoration. Cela représente donc une technologie très complexe à mettre en œuvre.

Il existe donc un réel besoin de mettre au point un procédé de production de HFO-1234yf dans lequel la réaction de fluoration peut être mise en œuvre à une pression modérée.

### RESUME DE L'INVENTION

L'invention concerne en premier lieu un procédé de production de 2,3,3,3-tétrafluoropropène comprenant :
- une réaction de fluoration catalytique en phase gazeuse d'un halopropane et / ou halopropène en 2,3,3,3-tétrafluoropropène au moyen de fluorure d'hydrogène ;
- la récupération d'un flux gazeux issu de la réaction ;
- le refroidissement et la condensation partielle du flux gazeux issu de la réaction, en un flux partiellement condensé ;
- la séparation du flux partiellement condensé en une fraction gazeuse et une fraction liquide ;
- la compression de la fraction gazeuse en une fraction gazeuse comprimée ;
- la distillation de la fraction gazeuse comprimée pour fournir un flux de 2,3,3,3-tétrafluoropropène, un flux d'acide chlorhydrique, et un flux de fluorure d'hydrogène non réagi.

Selon un mode de réalisation préféré, le halopropane et / ou halopropène est un chloropropane et / ou chloropropène, et de préférence est choisi parmi le 1,1,1,2,3-pentachloropropane, le 1,1,2,2,3-pentachloropropane, le 1,1,2,3-tétrachloropropène, le 2,3,3,3-tétrachloropropène, le 1,1,1-trifluoro-2-chloropropène et le 1,1,1-trifluoro-2,3-dichloropropane et les mélanges de ceux-ci.

Selon un mode de réalisation préféré, la séparation du flux partiellement condensé en une fraction gazeuse et une fraction liquide est effectuée par distillation.

Selon un mode de réalisation préféré, ledit procédé comprenant également une étape de compression de la fraction liquide en une fraction liquide comprimée.

Selon un mode de réalisation préféré, le flux de fluorure d'hydrogène non réagi est recyclé vers la réaction de fluoration.

Selon un mode de réalisation préféré, la réaction de fluoration est effectuée à une pression inférieure à celle de la distillation de la fraction gazeuse comprimée et de la fraction liquide comprimée.

Selon un mode de réalisation préféré, la réaction de fluoration est effectuée à une pression de 0,1 à 10 bar abs, et de préférence de 0,3 à 8 bar abs ; et / ou la distillation est effectuée à une pression de 5 à 40 bar abs, et plus préférentiellement de 7 à 25 bar abs.

La présente invention concerne également une installation de production de 2,3,3,3-tétrafluoropropène comprenant :
- au moins un réacteur de fluoration (6) alimenté en halopropane et / ou halopropène et en fluorure d'hydrogène ;
- une ligne (7) de gaz réactionnels, connectée en sortie du réacteur de fluoration (6) ;
- des moyens de refroidissement et de condensation partielle (8), alimentés par la ligne (7) de gaz réactionnels ;
- une ligne (9) de transport de flux partiellement condensé connectée en sortie des moyens de refroidissement et de condensation partielle (8) ;
- un moyen de séparation (10) alimenté par la ligne (9) de transport de flux partiellement condensé ;
- une ligne de soutirage de fraction gazeuse (11, 13) et une ligne de soutirage de fraction liquide (18) connectées en sortie du ballon de séparation (10) ;
- un compresseur (14) alimenté par la ligne de soutirage de fraction gazeuse (11, 13) ;
- une ligne d'alimentation en fraction gazeuse comprimée (15, 17) connectée en sortie du compresseur (14) ;
- des moyens de distillation (21, 23) alimentés par la ligne d'alimentation en fraction gazeuse comprimée (15, 17) ;
- une ligne de soutirage de 2,3,3,3-tétrafluoropropène (28), une ligne de soutirage d'acide chlorhydrique (26), et une ligne de soutirage de fluorure d'hydrogène non réagi (29).

Selon un mode de réalisation préféré, la ligne de soutirage de fluorure d'hydrogène non réagi est connectée au réacteur de fluoration.

Selon un mode de réalisation préféré, l'installation comprend également une pompe (19) alimentée par la ligne de soutirage de fraction liquide (18) ; et une ligne d'alimentation en fraction liquide comprimée (20) connectée en sortie de la pompe (19).

Selon un mode de réalisation préféré, la ligne d'alimentation en fraction liquide comprimée (20) alimente les moyens de distillation (21).

Selon un mode de réalisation, l'installation comprend des moyens de chauffage (12) sur la ligne de soutirage de fraction gazeuse (11, 13).

Selon un mode de réalisation préféré, la ligne d'alimentation en fraction gazeuse comprimée (15, 17) et la ligne d'alimentation en fraction liquide comprimée (20) alimentent la première colonne de distillation (21) à des étages différents de celle-ci.

Selon un mode de réalisation préféré, la ligne de soutirage de 2,3,3,3-tétrafluoropropène (28) alimente des moyens de purification supplémentaires, de préférence choisis parmi des moyens de lavage, d'extraction, de décantation et de distillation.

La présente invention permet de surmonter les inconvénients de l'état de la technique. Elle fournit plus particulièrement un procédé de production de HFO-1234yf dans lequel la réaction de fluoration peut être mise en œuvre à une pression modérée, et ce de manière simple et économique, et notamment sans avoir à recourir à un compresseur capable de comprimer un débit de gaz trop important.

Ceci est accompli en prévoyant un refroidissement et une condensation partielle des gaz issus de l'étape de réaction, puis une séparation de ces produits en une fraction liquide et une fraction gazeuse. De la sorte, seule une partie des gaz issus de la réaction (fraction gazeuse) doivent être comprimés dans un compresseur, tandis que la fraction liquide (gaz condensés) peut être reprise par une pompe, plus simple à mettre en œuvre qu'un compresseur.

En outre, la fraction gazeuse comporte avantageusement une faible teneur en HF, le HF se trouvant majoritairement dans la fraction liquide. La conception du compresseur s'en trouve simplifiée, notamment en termes de choix de matériaux, ce compresseur n'ayant pas à être en contact avec une quantité élevée de HF.

Ainsi, l'invention permet de pouvoir opérer la réaction de fluoration et la séparation des produits issus de la réaction à des pressions optimales et indépendantes.

Selon un mode de réalisation particulièrement avantageux, l'invention prévoit l'injection de la fraction liquide et de la fraction gazeuse à des étages différents d'une colonne de séparation, ce qui permet d'optimiser la conception de la colonne de séparation en termes de nombre d'étages théoriques et de taux de reflux à mettre en œuvre. La taille de la colonne et la puissance thermique nécessaire pour le condenseur et le rebouilleur de la colonne peuvent ainsi être minimisées, de même que la taille du groupe froid associé au condenseur.

### BREVE DESCRIPTION DES FIGURES

La **figure 1** représente de manière schématique une installation selon un premier mode de réalisation de l'invention.
La **figure 2** représente de manière schématique une installation selon un deuxième mode de réalisation de l'invention.

### DESCRIPTION DE MODES DE REALISATION DE L'INVENTION

L'invention est maintenant décrite plus en détail et de façon non limitative dans la description qui suit.

### Réaction de fluoration

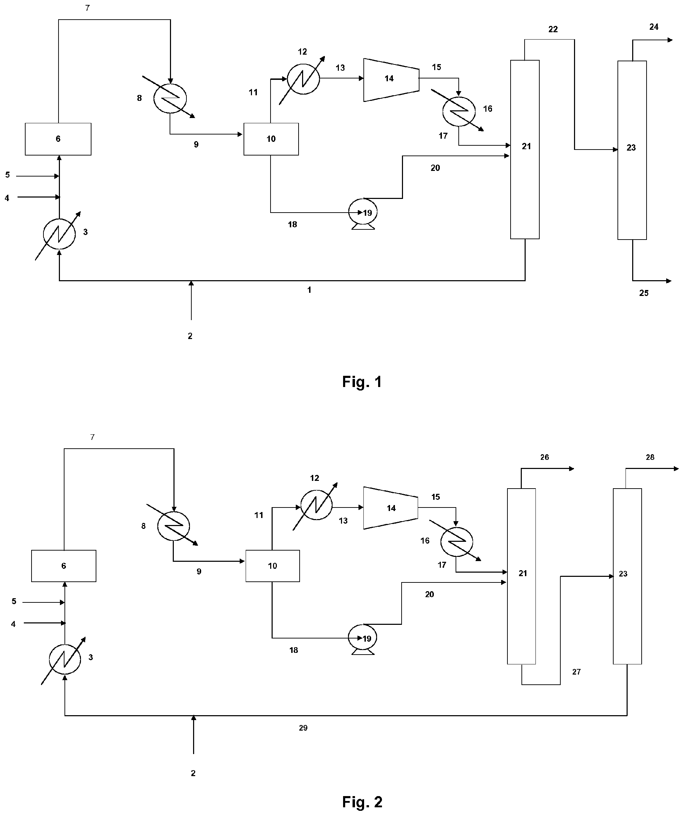

Selon un premier mode de réalisation, et en faisant référence à la **figure 1****,** une installation selon l'invention comporte généralement un réacteur de fluoration 6. On peut également utiliser deux ou plus de deux réacteurs disposés en série. Ce réacteur de fluoration est alimenté en halopropane et / ou halopropène par une ligne d'alimentation en hydrohalogénocarbure 4, et en HF par une ligne d'alimentation en HF 2.

A titre d'halopropane ou d'halopropène, on peut notamment utiliser tout chlorofluoropropane et / ou chlorofluoropropène, ou, selon un mode de réalisation préféré, tout chloropropane et / ou chloropropène.

Par exemple, les halopropanes de formule CX₃CHClCH₂X ou de formule CHX₂CClXCH₂X, les halopropènes de formule CX₃CCl=CH₂ ou de formule CX₂=CClCH₂X, chaque X représentant F ou Cl de manière indépendante, peuvent convenir.

Des halopropanes et halopropènes préférés sont le HCC-240db, le HCC-240aa, le HCO-1230xa et le HCO-1230xf, ou encore le HCFO-1233xf et le HCFC-243db (2,3-dichloro-1,1,1-trifluoropropane). Des mélanges de ces composés sont également possibles.

La suite de la description est effectuée en relation avec le HCC-240db, étant entendu que les autres halopropanes et / halopropènes peuvent être utilisés de façon analogue.

Le HCC-240db est converti en HFO-1234yf par réaction avec HF dans le réacteur de fluoration 6. La teneur en HFO-1234yf dans le flux issu de la réaction est de préférence d'au moins 0,5 mol%, notamment d'au moins 1 mol%, et plus préférentiellement d'au moins 2 mol% ou au moins 3 mol%.

Outre le HCl, d'autres composés sont formés au cours de la réaction, par exemple le HCFO-1233xf et le HFC-245cb (1,1,1,2,2-pentafluoropropane) dans le cas d'une réaction à partir du HCC-240db.

Le réacteur de fluoration 6 est un réacteur catalytique contenant un catalyseur.

Le catalyseur est par exemple basé sur un métal comprenant un oxyde de métal de transition ou un dérivé ou un halogénure ou un oxyhalogénure d'un tel métal. Des exemples de catalyseurs sont FeCl₃, l'oxyfluorure de chrome, les oxydes de chrome (éventuellement soumis à des traitements de fluoration), les fluorures de chrome et les mélanges de ceux-ci. D'autres catalyseurs possibles sont les catalyseurs supportés sur du carbone qui sont basés sur l'antimoine et sur l'aluminium (AlF₃, Al₂O₃, oxyfluorure d'alumine, fluorure d'aluminium). De manière générale, des catalyseurs pouvant être utilisés sont l'oxyfluorure de chrome, le fluorure et l'oxyfluorure d'aluminium et les catalyseurs supportés ou non contenant un métal tel que Cr, Ni, Zn, Ti, V, Zr, Mo, Ge, Sn, Pb, Mg. On peut également faire référence aux documents WO 2007/079431 en p.7, I.1-5 et 28-32, EP 0939071 au paragraphe [0022], WO 2008/054781 en p.9 I.22-p.10 I.34, WO 2008/040969 en revendication 1, tous incorporés ici par référence.

Avant utilisation, le catalyseur est soumis à une activation, typiquement avec de l'air, de l'oxygène, ou du chlore et/ou avec du HF, dans des conditions appropriées.

Dans un mode de réalisation préféré, on utilise un catalyseur mixte, contenant à la fois du chrome et du nickel. Le rapport molaire Cr:Ni, en éléments métalliques, est généralement de 0,5 à 5, par exemple de 0,7 à 2, par exemple d'environ 1. Le catalyseur peut comprendre, en masse, de 0,5 à 20 % de chrome et de 0,5 à 20 % de nickel, de préférence de 2 à 10 % de chaque métal.

Le métal peut être présent sous forme métallique ou sous forme d'un dérivé, notamment oxyde, halogénure ou oxyhalogénure. Ces dérivés, notamment halogénures et halogénures oxydes, sont obtenus par activation du métal catalytique. Bien que l'activation du métal ne soit pas nécessaire, elle est préférée.

Le support est de préférence préparé à partir d'aluminium, par exemple alumine, alumine activée ou dérivés d'aluminium. Ces dérivés comprennent les halogénures d'aluminium et les halogénures oxydes d'aluminium, par exemple tels que décrits dans le document US 4,902,838, ou obtenus par activation.

Le catalyseur peut comprendre du chrome et du nickel sous forme activée ou non activée, sur un support qui a été soumis à une activation ou non.

On peut faire référence au document WO 2009/118628, et particulièrement à la description du catalyseur de la p.4 I.30 à la p.7, I.16, qui est incorporé ici par référence.

Selon un autre mode de réalisation particulièrement préféré, le catalyseur peut aussi être du Cr à surface spécifique élevée, qui est de préférence non-supporté. Le catalyseur peut contenir une faible teneur en un ou plusieurs co-catalyseurs tels que les sels de Co, Zn, Mn, Mg et Ni. Un co-catalyseur préféré est Ni. Un autre co-catalyseur préféré est Zn. Un autre co-catalyseur préféré est Mg. Une description du catalyseur à base de Cr à surface spécifique élevée figure dans le document WO 2009/158321, en p. 4 et 6.

Le procédé selon l'invention est de préférence mis en œuvre de manière continue, ce qui est hautement avantageux sur le plan industriel.

En général, on utilise un rapport molaire de HF par rapport aux composés organiques (hydrocarbures et dérivés halogénés) qui est de 4:1 à 100:1, et de préférence de 5:1 à 50:1. L'utilisation de rapports surstœchiométriques pour la réaction de fluoration est enseignée par exemple dans les documents WO 2008/054781 et WO 2008/040969.

La réaction peut être mise en œuvre à une pression de 0,1 à 10 bar abs, et de préférence de 0,3 à 8 bar abs.

La réaction peut être mise en œuvre à une température de 100 à 500°C, de préférence de 200 à 450°C. La température du lit du réacteur peut être essentiellement uniforme ou peut varier le long du flux, en croissant ou en décroissant.

Le temps de contact (volume de catalyseur divisé par le débit total des réactifs et autres composés en entrée, ajusté selon la pression et la température de réaction), est typiquement de 1 à 100 s, de préférence de 5 à 50 s.

On peut prévoir aussi une alimentation en composé oxydant, notamment oxygène ou chlore, de préférence oxygène, afin de prolonger la durée de vie du catalyseur. Dans l'exemple illustré, le réacteur de fluoration 6 est alimenté en oxygène par une ligne d'alimentation en oxygène 5.

Le rapport molaire d'oxygène par rapport aux composés organiques est de préférence de 0,005 à 2 et plus préférentiellement de 0,01 à 1,5. L'oxygène peut être introduit sous forme quasi-pure ou bien sous forme d'air, ou bien sous forme de mélange azote / oxygène.

Un inhibiteur de polymérisation peut également être utilisé pour prolonger la durée de vie du catalyseur, typiquement à une concentration de 50 à 1000 ppm, plus préférentiellement de 100 à 500 ppm. On peut le choisir notamment parmi le p-méthoxyphénol, le t-amylphénol, le limonène, le d,1-limonène, les quinones, les hydroquinones, les époxydes, les amines et les mélanges de ceux-ci. Le p-méthoxyphénol et le t-amylphénol sont préférés. La co-alimentation en inhibiteur de polymérisation (non représentée sur les dessins) peut permettre de contrôler la polymérisation des chlorooléfines et ainsi notamment prolonger la durée de vie du catalyseur décrit dans le document US 5,714,651, incorporé ici par référence.

### Traitement du flux issu de la réaction de fluoration

Le flux issu de la réaction est récupéré dans une ligne 7 de gaz réactionnels connectée en sortie du réacteur de fluoration 6. Ce flux est refroidi, et partiellement condensé, par des moyens de refroidissement et de condensation partielle 8 (tels que des échangeurs et économiseurs) qui sont alimentés par la ligne 7 de gaz réactionnels.

La température du flux après refroidissement et condensation partielle est de -50 à 100°C, de préférence de -40 à 80°C. La température choisie dépend de la pression utilisée (du vide à environ 8 bar abs).

Une ligne 9 de transport de flux partiellement condensé est connectée en sortie des moyens de refroidissement et de condensation partielle 8. Elle transporte le flux issu de la réaction, après son refroidissement et sa condensation partielle.

La ligne 9 de transport de flux partiellement condensé alimente un ballon de séparation 10, qui permet de séparer le flux partiellement condensé en une fraction gazeuse et une fraction liquide. Le ballon de séparation 10 est un réservoir horizontal ou vertical apte à séparer physiquement les gaz des liquides, de préférence sans apport d'énergie.

La fraction liquide est récupérée en pied du ballon de séparation 10 et collectée par une ligne de soutirage de fraction liquide 18.

La fraction gazeuse est récupérée en tête du ballon de séparation 10 et collectée par une ligne de soutirage de fraction gazeuse 11, 13.

De préférence, la fraction gazeuse représente de 25 à 60 % du flux, et la phase liquide de 40 à 75 % du flux, en proportions massiques.

Des moyens de chauffage 12 sont prévus sur la ligne de soutirage de fraction gazeuse 11, 13, afin de réchauffer la fraction gazeuse et ainsi d'éviter toute condensation lors de la compression ultérieure.

La ligne de soutirage de fraction gazeuse 11, 13 alimente un compresseur 14. Le compresseur est fabriqué en matériaux résistants à la corrosion, tels que l'acier inoxydable 316L, l'Hastelloy® ou l'Inconel®.

En sortie du compresseur 14, la pression est de préférence de 5 à 40 bar abs, et plus préférentiellement de 7 à 25 bar abs.

La fraction gazeuse comprimée est collectée par une ligne d'alimentation en fraction gazeuse comprimée 15, 17 connectée en sortie du compresseur 14. Des moyens de refroidissement 16 peuvent être prévus sur la ligne d'alimentation en fraction gazeuse comprimée 15, 17, dans le but de refroidir et éventuellement condenser partiellement la fraction gazeuse comprimée avant les étapes de séparation et purification.

De préférence, la fraction gazeuse comprimée est ainsi refroidie à une température de -10 à 50°C.

La ligne de soutirage de fraction liquide 18 débouche sur une pompe 19. Cette pompe 19 permet d'acheminer la fraction liquide jusqu'aux moyens de séparation et purification, par une ligne d'alimentation en fraction liquide comprimée 20.

Les moyens de séparation et purification du flux issu de la réaction comprennent principalement des moyens de distillation.

Les moyens de distillation sont de préférence opérés à une pression de 5 à 40 bar abs, et plus préférentiellement de 7 à 25 bar abs

Dans le mode de réalisation illustré, les moyens de distillation comprennent une première colonne de distillation 21 et une deuxième colonne de distillation 23.

Chaque colonne de distillation est pourvue d'un rebouilleur en pied et d'un système de condensation et de reflux en tête, de manière connue en soi.

La première colonne de distillation 21 est alimentée par le flux issu de la réaction. De préférence, la fraction liquide comprimée (apportée par la ligne d'alimentation en fraction liquide comprimée 20) et la fraction gazeuse comprimée (apportée par la ligne d'alimentation en fraction gazeuse comprimée 15, 17) alimentent la première colonne de distillation 21 à des niveaux différents, notamment selon leurs compositions respectives.

La fonction de la première colonne de distillation 21 est de séparer le HCl et le HFO-1234yf d'une part, et le HF et composés organiques (chlorés et / ou fluorés) d'autre part.

Ainsi, une ligne de soutirage de flux intermédiaire 22 est connectée en tête de la première colonne de distillation 21, et une ligne de soutirage de fluorure d'hydrogène non réagi 1 est connectée en pied de la première colonne de distillation 21.

Le flux intermédiaire transporté dans la ligne de soutirage de flux intermédiaire 22 contient principalement du HCl, du HFO-1234yf, ainsi que des composés légers, à savoir azote, oxygène, monoxyde de carbone, dioxyde de carbone et autres dérivés oxygénés. Il peut également contenir du HFC-245cb, issu de la réaction de fluoration, qui pourra être ultérieurement recyclé au réacteur de fluoration, et une faible quantité de HF lié à l'existence d'azéotropes.

Le flux appelé ici « *flux de fluorure d'hydrogène non réagi* », transporté dans la ligne de soutirage de fluorure d'hydrogène non réagi 1, contient non seulement du HF mais également d'autres composés organiques chlorés et / ou fluorés, tels que le HCFO-1233xf et le HFC-245cb, qui sont des intermédiaires de réaction ou des composés organiques issus de réactions secondaires

Ce flux de fluorure d'hydrogène non réagi est recyclé vers le réacteur de fluoration 6. Des moyens de chauffage 3 (tels que des échangeurs et économiseurs) sont prévus sur la ligne de soutirage de fluorure d'hydrogène non réagi 1 afin de réchauffer et vaporiser le flux avant son entrée dans le réacteur de fluoration 6.

La ligne d'alimentation en hydrohalogénocarbure 4, la ligne d'alimentation en HF 2 et / ou la ligne d'alimentation en oxygène 5 peuvent être par exemple connectées à la ligne de soutirage de fluorure d'hydrogène non réagi 1, en amont et / ou en aval des moyens de chauffage 3, afin que le réacteur de fluoration 6 soit alimenté par une conduite unique. Alternativement, le réacteur de fluoration 6 peut être alimenté par les lignes respectives à différents endroits. On peut également prévoir une ligne de recyclage direct de la sortie du réacteur de fluoration 6 vers l'entrée (ou vers une entrée) de celui-ci, avec des moyens appropriés tels que des moyens de chauffage, de refroidissement et / ou de compression.

Un couplage énergétique peut être prévu entre les moyens de chauffage 3 et les moyens de refroidissement et de condensation partielle 8, afin que les calories récupérées lors du refroidissement et de la condensation partielle du flux gazeux issu de la réaction soient réemployées pour le chauffage et la vaporisation du flux en entrée du réacteur de fluoration 6.

Le flux intermédiaire fait l'objet d'une séparation dans la deuxième colonne de distillation 23, afin de récupérer d'une part le produit d'intérêt HFO-1234yf, et d'autre part le HCl avec les composés légers. A cet effet, la deuxième colonne de distillation 23 est alimentée en entrée par la ligne de soutirage de flux intermédiaire 22. En sortie de cette deuxième colonne de distillation 23 sont connectées, en tête, une ligne de soutirage d'acide chlorhydrique 24 ; et, en pied, une ligne de soutirage de 2,3,3,3-tétrafluoropropène 25.

Le flux de HFO-1234yf récupéré en pied peut contenir du HFC-245cb, ainsi qu'une faible quantité de HF.

Le flux de HCl récupéré en tête contient en général également les composés légers O₂, N₂, CO₂ et CO (et / ou autres dérivés oxygénés).

Des étapes supplémentaires de purification du flux de HFO-1234yf peuvent être mises en œuvre si nécessaire, reposant par exemple sur un lavage, une extraction, une décantation, une distillation ou une combinaison de ces opérations.

Le HF et les composés organiques chlorés ou fluorés différents du HFO-1234yf qui sont récupérés lors de ces étapes supplémentaires sont de préférence recyclés vers le réacteur de fluoration 6 (non représenté).

Un deuxième mode de réalisation du traitement du flux issu de la réaction est illustrée à la **figure 2****.** Selon cette variante, la fonction de la première colonne de distillation 21 est de séparer le HCl d'une part, et le HFO-1234yf, le HF et les composés organiques (chlorés et / ou fluorés) d'autre part.

Ainsi, une ligne de soutirage de flux intermédiaire 27 est connectée en pied de la première colonne de distillation 21, et une ligne de soutirage d'acide chlorhydrique 26 est connectée en tête de la première colonne de distillation 21.

Le flux de HCl récupéré en tête contient en général également les composés légers O₂, N₂, CO₂ et CO (et / ou autres dérivés oxygénés).

Le flux intermédiaire transporté dans la ligne de soutirage de flux intermédiaire 27 contient principalement du HFO-1234yf, du HF, ainsi que d'autres composés organiques chlorés et / ou fluorés, tels que du HFC-245cb ou du HCFO-1233xf.

Le flux intermédiaire fait l'objet d'une séparation dans la deuxième colonne de distillation 23, afin de récupérer d'une part le produit d'intérêt HFO-1234yf, et d'autre part les autres composés organiques chlorés et / ou fluorés. A cet effet, la deuxième colonne de distillation 23 est alimentée en entrée par la ligne de soutirage de flux intermédiaire 27. En sortie de cette deuxième colonne de distillation 23 sont connectées, en tête, une ligne de soutirage de 2,3,3,3-tétrafluoropropène 28 ; et, en pied, une ligne de soutirage de flux de fluorure d'hydrogène non réagi 29.

Le flux de HFO-1234yf récupéré en tête peut contenir du HFC-245cb, ainsi qu'une faible quantité de HF.

Le flux de fluorure d'hydrogène non réagi, transporté dans la ligne de soutirage de fluorure d'hydrogène non réagi 29 contient non seulement du HF, mais également d'autres composés organiques chlorés et / ou fluorés, tels que le HCFO-1233xf et le HFC-245cb, qui sont des intermédiaires de la réaction de fluoration ou des composés organiques issus de réactions secondaires (tout comme pour le premier mode de réalisation). Il est recyclé vers le réacteur de fluoration 6 de la même manière que pour le premier mode de réalisation.

Parmi les nombreuses variantes possibles de l'invention, on peut notamment citer la possibilité de remplacer les équipements uniques par une pluralité d'équipements fonctionnant en parallèle ou en série, par exemple plusieurs réacteurs de fluoration, et / ou plusieurs premières colonnes de distillation, et / ou plusieurs deuxièmes colonnes de distillation. Les lignes connectant les différents équipements sont adaptées en conséquence.
Mode de réalisation 1 : Procédé de production de 2,3,3,3-tétrafluoropropène comprenant :
   - une réaction de fluoration d'un halopropane et / ou halopropène en 2,3,3,3-tétrafluoropropène au moyen de fluorure d'hydrogène ;
   - la récupération d'un flux gazeux issu de la réaction ;
   - le refroidissement et la condensation partielle du flux gazeux issu de la réaction, en un flux partiellement condensé ;
   - la séparation du flux partiellement condensé en une fraction gazeuse et une fraction liquide ;
   - la compression de la fraction gazeuse en une fraction gazeuse comprimée ;
   - la compression de la fraction liquide en une fraction liquide comprimée
   - la distillation de la fraction gazeuse comprimée et de la fraction liquide comprimée pour fournir un flux de 2,3,3,3-tétrafluoropropène, un flux d'acide chlorhydrique, et un flux de fluorure d'hydrogène non réagi.
Mode de réalisation 2 : Procédé selon le mode de réalisation 1, dans lequel le flux de fluorure d'hydrogène non réagi comprend également des composés organiques intermédiaires de la réaction de fluoration et / ou des composés organiques issus de réactions secondaires.
Mode de réalisation 3 : Procédé selon le mode de réalisation 1 ou 2, dans lequel le flux de fluorure d'hydrogène non réagi est recyclé vers la réaction de fluoration.
Mode de réalisation 4 : Procédé selon l'un des modes de réalisation 1 à 3, dans lequel la réaction de fluoration est une réaction de fluoration catalytique en phase gazeuse.
Mode de réalisation 5 : Procédé selon l'un des modes de réalisation 1 à 4, dans lequel la réaction de fluoration est effectuée à une pression inférieure à celle de la distillation de la fraction gazeuse comprimée et de la fraction liquide comprimée.
Mode de réalisation 6 : Procédé selon l'un des modes de réalisation 1 à 5, dans lequel la réaction de fluoration est effectuée à une pression de 0,1 à 10 bar abs, et de préférence de 0,3 à 8 bar abs ; et / ou la distillation est effectuée à une pression de 5 à 40 bar abs, et plus préférentiellement de 7 à 25 bar abs.
Mode de réalisation 7 : Procédé selon l'un des modes de réalisation 1 à 6, dans lequel la fraction gazeuse représente de 25 à 60 % du flux partiellement condensé, et la fraction liquide représente de 40 à 75 % du flux partiellement condensé, en proportions massiques.
Mode de réalisation 8 : Procédé selon l'un des modes de réalisation 1 à 7, dans lequel l'étape de distillation comprend :
   - une première distillation de la fraction gazeuse comprimée et de la fraction liquide comprimée ;
   - la récupération du flux de fluorure d'hydrogène non réagi à l'issue de la première distillation ;
   - la récupération d'un flux intermédiaire à l'issue de la première distillation ;
   - une deuxième distillation du flux intermédiaire ;
   - la récupération du flux d'acide chlorhydrique à l'issue de la deuxième distillation ; et
   - la récupération du flux de 2,3,3,3-tétrafluoropropène à l'issue de la deuxième distillation.
Mode de réalisation 9 : Procédé selon l'un des modes de réalisation 1 à 7, dans lequel l'étape de distillation comprend :
   - une première distillation de la fraction gazeuse comprimée et de la fraction liquide comprimée ;
   - la récupération du flux d'acide chlorhydrique à l'issue de la première distillation ;
   - la récupération d'un flux intermédiaire à l'issue de la première distillation ;
   - une deuxième distillation du flux intermédiaire ;
   - la récupération du flux de 2,3,3,3-tétrafluoropropène à l'issue de la deuxième distillation ; et
   - la récupération du flux de fluorure d'hydrogène non réagi à l'issue de la deuxième distillation.
Mode de réalisation 10 : Procédé selon l'un des modes de réalisation 1 à 9, dans lequel la fraction gazeuse comprimée et la fraction liquide comprimée sont introduites à des emplacements différents d'une colonne de distillation.
Mode de réalisation 11 : Procédé selon l'un des modes de réalisation 1 à 10, dans lequel le flux de 2,3,3,3-tétrafluoropropène subit une ou plusieurs étapes de purification supplémentaires, de préférence choisies parmi un lavage, une extraction, une décantation et une distillation.
Mode de réalisation 12 : Procédé selon l'un des modes de réalisation 1 à 11, dans lequel le halopropane et / ou halopropène est un chloropropane et / ou chloropropène, et de préférence est choisi parmi le 1,1,1,2,3-pentachloropropane, le 1,1,2,2,3-pentachloropropane, le 1,1,2,3-tétrachloropropène, le 2,3,3,3-tétrachloropropène, le 1,1,1-trifluoro-2-chloropropène et le 1,1,1-trifluoro-2,3-dichloropropane et les mélanges de ceux-ci.
Mode de réalisation 13 : Installation de production de 2,3,3,3-tétrafluoropropène comprenant :
   - au moins un réacteur de fluoration (6) alimenté en halopropane et / ou halopropène et en fluorure d'hydrogène ;
   - une ligne (7) de gaz réactionnels, connectée en sortie du réacteur de fluoration (6) ;
   - des moyens de refroidissement et de condensation partielle (8), alimentés par la ligne (7) de gaz réactionnels ;
   - une ligne (9) de transport de flux partiellement condensé connectée en sortie des moyens de refroidissement et de condensation partielle (8) ;
   - un ballon de séparation (10) alimenté par la ligne (9) de transport de flux partiellement condensé ;
   - une ligne de soutirage de fraction gazeuse (11, 13) et une ligne de soutirage de fraction liquide (18) connectées en sortie du ballon de séparation (10) ;
   - un compresseur (14) alimenté par la ligne de soutirage de fraction gazeuse (11, 13) ;
   - une ligne d'alimentation en fraction gazeuse comprimée (15, 17) connectée en sortie du compresseur (14) ;
   - une pompe (19) alimentée par la ligne de soutirage de fraction liquide (18) ;
   - une ligne d'alimentation en fraction liquide comprimée (20) connectée en sortie de la pompe (19) ;
   - des moyens de distillation (21, 23) alimentés par la ligne d'alimentation en fraction gazeuse comprimée (15, 17) et par la ligne d'alimentation en fraction liquide comprimée (20) ;
   - une ligne de soutirage de 2,3,3,3-tétrafluoropropène (25, 28), une ligne de soutirage d'acide chlorhydrique (24, 26), et une ligne de soutirage de fluorure d'hydrogène non réagi (1, 29), connectées en sortie des moyens de distillation (21, 23).
Mode de réalisation 14 : Installation selon le mode de réalisation 13, dans laquelle la ligne de soutirage de fluorure d'hydrogène non réagi (1, 29) alimente le réacteur de fluoration (6).
Mode de réalisation 15 : Installation selon le mode de réalisation 13 ou 14, comprenant des moyens de chauffage (12) sur la ligne de soutirage de fraction gazeuse (11, 13).
Mode de réalisation 16 : Installation selon l'un des modes de réalisation 13 à 15, dans laquelle les moyens de distillation (21, 23) comprennent une première colonne de distillation (21) et une deuxième colonne de distillation (23), la ligne d'alimentation en fraction gazeuse comprimée (15, 17) et la ligne d'alimentation en fraction liquide comprimée (20) alimentant la première colonne de distillation (21).
Mode de réalisation 17 : Installation selon le mode de réalisation 16, dans laquelle :
   - la ligne de soutirage de fluorure d'hydrogène non réagi (1) est connectée en pied de la première colonne de distillation (21) ;
   - une ligne de soutirage de flux intermédiaire (22) est connectée en tête de la première colonne de distillation (21) et alimente la deuxième colonne de distillation (23) ;
   - la ligne de soutirage d'acide chlorhydrique (24) est connectée en tête de la deuxième colonne de distillation (23) ; et
   - la ligne de soutirage de 2,3,3,3-tétrafluoropropène (25) est connectée en pied de la deuxième colonne de distillation (23).
Mode de réalisation 18 : Installation selon le mode de réalisation 16, dans laquelle :
   - la ligne de soutirage d'acide chlorhydrique (26) est connectée en tête de la première colonne de distillation (21) ;
   - une ligne de soutirage de flux intermédiaire (27) est connectée en pied de la première colonne de distillation (21) et alimente la deuxième colonne de distillation (23) ;
   - la ligne de soutirage de 2,3,3,3-tétrafluoropropène (28) est connectée en tête de la deuxième colonne de distillation (23) ; et
   - la ligne de soutirage de fluorure d'hydrogène non réagi (29) est connectée en pied de la deuxième colonne de distillation (23).
Mode de réalisation 19 : Installation selon l'un des modes de réalisation 13 à 18, dans laquelle la ligne d'alimentation en fraction gazeuse comprimée (15, 17) et la ligne d'alimentation en fraction liquide comprimée (20) alimentent la première colonne de distillation (21) à des étages différents de celle-ci.
Mode de réalisation 20 : Installation selon l'un des modes de réalisation 13 à 19, dans laquelle la ligne de soutirage de 2,3,3,3-tétrafluoropropène (25, 28) alimente des moyens de purification supplémentaires, de préférence choisis parmi des moyens de lavage, d'extraction, de décantation et de distillation.

## Revendications

1. Procédé de production de 2,3,3,3-tétrafluoropropène comprenant :
- une réaction de fluoration catalytique en phase gazeuse d'un halopropane et / ou halopropène en 2,3,3,3-tétrafluoropropène au moyen de fluorure d'hydrogène ;
- la récupération d'un flux gazeux issu de la réaction ;
- le refroidissement et la condensation partielle du flux gazeux issu de la réaction, en un flux partiellement condensé ;
- la séparation du flux partiellement condensé en une fraction gazeuse et une fraction liquide ;
- la compression de la fraction gazeuse en une fraction gazeuse comprimée ;
- la distillation de la fraction gazeuse comprimée pour fournir un flux comprenant d'une part HCl et d'autre part 2,3,3,3-tétrafluoropropène, HF n'ayant pas réagi, 2-chloro-3,3,3-trifluoropropène et 1,1,1,2,2-pentafluoropropane.

2. Procédé selon la revendication 1, dans lequel le halopropane et / ou halopropène est un chloropropane et / ou chloropropène, et de préférence est choisi parmi le 1,1,1,2,3-pentachloropropane, le 1,1,2,2,3-pentachloropropane, le 1,1,2,3-tétrachloropropène, le 2,3,3,3-tétrachloropropène, le 1,1,1-trifluoro-2-chloropropène et le 1,1,1-trifluoro-2,3-dichloropropane et les mélanges de ceux-ci.

3. Procédé selon la revendication 1 ou la revendication 2, dans lequel la séparation du flux partiellement condensé en une fraction gazeuse et une fraction liquide est effectuée par distillation.

4. Procédé selon l'une des revendications 1 à 3, ledit procédé comprenant également une étape de compression de la fraction liquide en une fraction liquide comprimée.

5. Procédé selon l'une des revendications 1 à 4, dans lequel le flux de fluorure d'hydrogène non réagi est recyclé vers la réaction de fluoration.

6. Procédé selon l'une des revendications 1 à 5, dans lequel la réaction de fluoration est effectuée à une pression inférieure à celle de la distillation de la fraction gazeuse comprimée et de la fraction liquide comprimée.

7. Procédé selon l'une des revendications 1 à 6, dans lequel la réaction de fluoration est effectuée à une pression de 0,1 à 10 bar abs, et de préférence de 0,3 à 8 bar abs ; et / ou la distillation est effectuée à une pression de 5 à 40 bar abs, et plus préférentiellement de 7 à 25 bar abs.

8. Installation de production de 2,3,3,3-tétrafluoropropène comprenant :
- au moins un réacteur de fluoration (6) alimenté en halopropane et / ou halopropène et en fluorure d'hydrogène ;
- une ligne (7) de gaz réactionnels, connectée en sortie du réacteur de fluoration (6) ;
- des moyens de refroidissement et de condensation partielle (8), alimentés par la ligne (7) de gaz réactionnels ;
- une ligne (9) de transport de flux partiellement condensé connectée en sortie des moyens de refroidissement et de condensation partielle (8) ;
- un moyen de séparation (10) alimenté par la ligne (9) de transport de flux partiellement condensé ;
- une ligne de soutirage de fraction gazeuse (11, 13) et une ligne de soutirage de fraction liquide (18) connectées en sortie du ballon de séparation (10) ;
- un compresseur (14) alimenté par la ligne de soutirage de fraction gazeuse (11, 13) ;
- une ligne d'alimentation en fraction gazeuse comprimée (15, 17) connectée en sortie du compresseur (14) ;
- des moyens de distillation (21, 23) alimentés par la ligne d'alimentation en fraction gazeuse comprimée (15, 17) ;
- une ligne de soutirage de 2,3,3,3-tétrafluoropropène (28), une ligne de soutirage d'acide chlorhydrique (26), et une ligne de soutirage de fluorure d'hydrogène non réagi (29).

9. Installation selon la revendication 8 **caractérisé en ce que** la ligne de soutirage de fluorure d'hydrogène non réagi est connectée au réacteur de fluoration.

10. Installation selon la revendication 8 ou la revendication 9 **caractérisé en ce qu'**elle comprend également une pompe (19) alimentée par la ligne de soutirage de fraction liquide (18) ; et une ligne d'alimentation en fraction liquide comprimée (20) connectée en sortie de la pompe (19).

11. Installation selon l'une quelconque des revendications 8 à 10 **caractérisé en ce que** la ligne d'alimentation en fraction liquide comprimée (20) alimente les moyens de distillation (21).

12. Installation selon l'une quelconque des revendications 8 à 11, comprenant des moyens de chauffage (12) sur la ligne de soutirage de fraction gazeuse (11, 13).

13. Installation selon l'une des revendications 8 à 12, dans laquelle la ligne d'alimentation en fraction gazeuse comprimée (15, 17) et la ligne d'alimentation en fraction liquide comprimée (20) alimentent la première colonne de distillation (21) à des étages différents de celle-ci.

14. Installation selon l'une des revendications 8 à 13, dans laquelle la ligne de soutirage de 2,3,3,3-tétrafluoropropène (28) alimente des moyens de purification supplémentaires, de préférence choisis parmi des moyens de lavage, d'extraction, de décantation et de distillation.

## Patentansprüche

1. Verfahren zur Herstellung von 2,3,3,3-Tetrafluorpropen, umfassend:
- eine katalytische Gasphasen-Fluorierungsreaktion eines Halogenpropans und/oder Halogenpropens zu 2,3,3,3-Tetrafluorpropen mit Hilfe von Fluorwasserstoff;
- das Gewinnen eines gasförmigen Stroms aus der Reaktion;
- das Abkühlen und teilweise Kondensieren des gasförmigen Stroms aus der Reaktion zu einem teilweise kondensierten Strom;
- das Trennen des teilweise kondensierten Stroms in eine gasförmige Fraktion und eine flüssige Fraktion;
- das Komprimieren der gasförmigen Fraktion zu einer komprimierten gasförmigen Fraktion;
- das Destillieren der komprimierten gasförmigen Fraktion zum Erhalt eines Stroms, der einerseits HCl und andererseits 2,3,3,3-Tetrafluorpropen, nicht umgesetztes HF, 2-Chlor-3,3,3-trifluorpropen und 1,1,1,2,2-Pentafluorpropan umfasst.

2. Verfahren nach Anspruch 1, bei dem das Halogenpropan und/oder Halogenpropen ein Chlorpropan und/oder Chlorpropen ist und vorzugsweise aus 1,1,1,2,3-Pentachlorpropan, 1,1,2,2,3-Pentachlorpropan, 1,1,2,3-Tetrachlorpropen, 2,3,3,3-Tetrachlorpropen, 1,1,1-Trifluor-2-chlorpropen und 1,1,1-Trifluor-2,3-dichlorpropan und Mischungen davon ausgewählt ist.

3. Verfahren nach Anspruch 1 oder Anspruch 2, bei dem die Trennung des teilweise kondensierten Stroms in eine gasförmige Fraktion und eine flüssige Fraktion mittels Destillation durchgeführt wird.

4. Verfahren nach einem der Ansprüche 1 bis 3, bei dem das Verfahren außerdem einen Schritt des Komprimierens der flüssigen Fraktion zu einer komprimierten flüssigen Fraktion umfasst.

5. Verfahren nach einem der Ansprüche 1 bis 4, bei dem der Strom von nicht umgesetztem Fluorwasserstoff zur Fluorierungsreaktion zurückgeführt wird.

6. Verfahren nach einem der Ansprüche 1 bis 5, bei dem die Fluorierungsreaktion bei einem Druck durchgeführt wird, der unter dem Druck der Destillation der komprimierten gasförmigen Fraktion, und der komprimierten flüssigen Fraktion liegt.

7. Verfahren nach einem der Ansprüche 1 bis 6, bei dem die Fluorierungsreaktion bei einem Druck von 0,1 bis 10 bar abs und vorzugsweise von 0,3 bis 8 bar abs durchgeführt wird und/oder die Destillation bei einem Druck von 5 bis 40 bar abs und weiter bevorzugt von 7 bis 25 bar abs durchgeführt wird.

8. Anlage zur Herstellung von 2,3,3,3-Tetrafluorpropen, umfassend:
- mindestens einen Fluorierungsreaktor (6), der mit Halogenpropan und/oder Halogenpropen und mit Fluorwasserstoff gespeist wird;
- eine Leitung (7) für Reaktionsgase, die mit dem Ausgang des Fluorierungsreaktors (6) verbunden ist;
- Einrichtungen zur Abkühlung und teilweisen Kondensation (8), die durch die Leitung (7) für Reaktionsgase gespeist werden;
- eine Leitung (9) zum Transport von teilweise kondensiertem Strom, die mit dem Ausgang der Einrichtungen zur Abkühlung und teilweisen Kondensation (8) verbunden sind;
- eine Trenneinrichtung (10), die durch die Leitung (9) zum Transport von teilweise kondensiertem Strom gespeist wird;
- eine Leitung zum Abziehen von gasförmiger Fraktion (11, 13) und eine Leitung zum Abziehen von flüssiger Fraktion (18), die mit dem Ausgang des Trennbehälters (10) verbunden sind;
- einen Verdichter (14), der durch die Leitung zum Abziehen von gasförmiger Fraktion (11, 13) gespeist wird;
- eine Leitung zum Einspeisen von komprimierter gasförmiger Fraktion (15, 17), die mit dem Ausgang des Verdichters (14) verbunden ist;
- Destillationseinrichtungen (21, 23), die durch die Leitung zum Einspeisen von komprimierter gasförmiger Fraktion (15, 17) gespeist werden;
- eine Leitung zum Abziehen von 2,3,3,3-Tetrafluorpropen (28), eine Leitung zum Abziehen von Salzsäure (26) und eine Leitung zum Abziehen von nicht umgesetztem Fluorwasserstoff (29).

9. Anlage nach Anspruch 8, **dadurch gekennzeichnet, dass** die Leitung zum Abziehen von nicht umgesetztem Fluorwasserstoff mit dem Fluorierungsreaktor verbunden ist.

10. Anlage nach Anspruch 8 oder Anspruch 9, **dadurch gekennzeichnet, dass** sie außerdem eine Pumpe (19), die durch die Leitung zum Abziehen von flüssiger Fraktion (18) gespeist wird, und eine Leitung zum Einspeisen von komprimierter flüssiger Fraktion (20), die mit dem Ausgang der Pumpe (19) verbunden ist, umfasst.

11. Anlage nach einem der Ansprüche 8 bis 10, **dadurch gekennzeichnet, dass** die Leitung zum Einspeisen von komprimierter flüssiger Fraktion (20) die Destillationseinrichtungen (21) speist.

12. Anlage nach einem der Ansprüche 8 bis 11, umfassend Heizeinrichtungen (12) an der Leitung zum Abziehen von gasförmiger Fraktion (11, 13).

13. Anlage nach einem der Ansprüche 8 bis 12, wobei die Leitung zum Einspeisen von komprimierter gasförmiger Fraktion (15, 17) und die Leitung zum Einspeisen von komprimierter flüssiger Fraktion (20) die erste Destillationssäule (21) auf verschiedenen Stufen davon speisen.

14. Anlage nach einem der Ansprüche 8 bis 13, wobei die Leitung zum Abziehen von 2,3,3,3-Tetrafluorpropen (28) zusätzliche Reinigungseinrichtungen, vorzugsweise ausgewählt aus Wasch-, Extraktions-, Absetz- und Destillationseinrichtungen, speist.

## Claims

1. Process for the production of 2,3,3,3-tetrafluoropropene, comprising:
- a gas-phase catalytic fluorination reaction of a halopropane and/or halopropene to give 2,3,3,3-tetrafluoropropene by means of hydrogen fluoride;
- the recovery of a gas stream resulting from the reaction;
- the cooling and the partial condensation of the gas stream resulting from the reaction to give a partially condensed stream;
- the separation of the partially condensed stream into a gas fraction and a liquid fraction;
- the compression of the gas fraction to give a compressed gas fraction;
- the distillation of the compressed gas fraction in order to provide a stream comprising, on the one hand, HCl and, on the other hand, 2,3,3,3-tetrafluoropropene, unreacted HF, 2-chloro-3,3,3-trifluoropropene and 1,1,1,2,2-pentafluoropropane.

2. Process according to Claim 1, in which the halopropane and/or halopropene is a chloropropane and/or chloropropene and is preferably chosen from 1,1,1,2,3-pentachloropropane, 1,1,2,2,3-pentachloropropane, 1,1,2,3-tetrachloropropene, 2,3,3,3-tetrachloropropene, 1,1,1-trifluoro-2-chloropropene and 1,1,1-trifluoro-2,3-dichloropropane and the mixtures of these.

3. Process according to Claim 1 or Claim 2, in which the separation of the partially condensed stream into a gas fraction and a liquid fraction is carried out by distillation.

4. Process according to one of Claims 1 to 3, said process also comprising a step of compression of the liquid fraction to give a compressed liquid fraction.

5. Process according to one of Claims 1 to 4, in which the unreacted hydrogen fluoride stream is recycled to the fluorination reaction.

6. Process according to one of Claims 1 to 5, in which the fluorination reaction is carried out at a lower pressure than that of the distillation of the compressed gas fraction and of the compressed liquid fraction.

7. Process according to one of Claims 1 to 6, in which the fluorination reaction is carried out at a pressure of 0.1 to 10 bar abs and preferably of 0.3 to 8 bar abs and/or the distillation is carried out at a pressure of 5 to 40 bar abs and more preferably of 7 to 25 bar abs.

8. Plant for the production of 2,3,3,3-tetrafluoropropene, comprising:
- at least one fluorination reactor (6) fed with halopropane and/or halopropene and with hydrogen fluoride;
- a line (7) for reaction gases, connected at the outlet of the fluorination reactor (6);
- cooling and partial condensation means (8), fed by the line (7) for reaction gases;
- a line (9) for transportation of partially condensed stream connected at the outlet of the cooling and partial condensation means (8);
- a separation means (10) fed by the line (9) for transportation of partially condensed stream;
- a gas fraction withdrawal line (11, 13) and a liquid fraction withdrawal line (18) which are connected at the outlet of the knockout vessel (10) ;
- a compressor (14) fed by the gas fraction withdrawal line (11, 13);
- a line for feeding with compressed gas fraction (15, 17) connected at the outlet of the compressor (14) ;
- distillation means (21, 23) fed by the line for feeding with compressed gas fraction (15, 17);
- a 2,3,3,3-tetrafluoropropene withdrawal line (28), a hydrochloric acid withdrawal line (26) and an unreacted hydrogen fluoride withdrawal line (29).

9. Plant according to Claim 8, **characterized in that** the unreacted hydrogen fluoride withdrawal line is connected to the fluorination reactor.

10. Plant according to Claim 8 or Claim 9, **characterized in that** it also comprises a pump (19) fed by the liquid fraction withdrawal line (18) and a line for feeding with compressed liquid fraction (20) connected at the outlet of the pump (19).

11. Plant according to any one of Claims 8 to 10, **characterized in that** the line for feeding with compressed liquid fraction (20) feeds the distillation means (21).

12. Plant according to any one of Claims 8 to 11, comprising heating means (12) on the gas fraction withdrawal line (11, 13).

13. Plant according to one of Claims 8 to 12, in which the line for feeding with compressed gas fraction (15, 17) and the line for feeding with compressed liquid fraction (20) feed the first distillation column (21) at different stages of the latter.

14. Plant according to one of Claims 8 to 13, in which the 2,3,3,3-tetrafluoropropene withdrawal line (28) feeds additional purification means, preferably chosen from washing, extraction, separation by settling and distillation means.
